# EUROPEAN PATENT SPECIFICATION

(11) **EP 0 632 720 B1**
(45) Date of publication and mention of the grant of the patent: **11.11.1998**
(21) Application number: 93907548.7
(22) Date of filing: 17.03.1993
(51) Int. Cl.: A61K 9/16

(54) **HYDROXYETHYLCELLULOSE-BASED SUSTAINED-RELEASE ORAL DRUG DOSAGE FROMS**
AUF HYDROXYETHYLZELLULOSE BASIERENDE ORALEN ARZNEIDOSISFORMEN MIT VERZOEGERTER WIRKSTOFFABGABE
FORMES GALENIQUES DE MEDICAMENT ORAL A LIBERATION PROLONGEE A BASE D'HYDROXYETHYL-CELLULOSE

(30) Priority: 25.03.1992 US 858320; 08.12.1992 US 986952
(43) Date of publication of application: 11.01.1995
(73) Proprietor: DEPOMED SYSTEMS, INC., Foster City, CA 94404 (US)
(72) Inventor: SHELL, John, W., Hillsborough, CA 94010 (US)
(74) Representative: Baldock, Sharon Claire
(86) International application number: US9302420
(87) International publication number: WO9318755

(56) References cited:
- WO-A-90/11757
- WO-A-92/04013
- DE-U- 9 107 805
- FR-A- 2 304 354
- FR-A- 2 417 982

## Description

This invention is in the general field of pharmacology and relates specifically to alkyl-substituted cellulose-based sustained-release drug dosage forms whose rate of drug release and dissolution is not dependent upon crosslinking and that may be made by direct compression and other procedures without binders.

It is known to use hydrophilic polymers such as hydroxyalkyl celluloses in the preparation of sustained release pharmaceutical compositions for oral administration. Such use is described for example in FR-A-2304354 and WO 92/04013.

The present invention is an improvement on the sustained-release oral drug dosage forms described in U.S. Patent No 5,007,790. Those dosage forms consist of a plurality of solid particles composed of a solid drug dispersed in a hydrophilic water-swellable crosslinked polymer. The polymers of the particle imbibe water, causing the particles to swell and the drug contained in the particle to dissolve and leach from the particle. After the drug has leached from the particles, the crosslinks of the polymer cleave to allow the polymer to dissolve.

In contrast to the polymers described in U.S. Patent No. 5,007,790, the polymers used in the present invention are not crosslinked. They are thus inherently safer in that possible toxicity from any residual crosslinking agent is avoided. In addition, the particles made from the present polymers may be formed into solid bodies (e.g., tablets) by direct compression without binders. Binders had to be added to the polymers of the prior patent in order to compression-mold the particles. This lack of binder makes the dosage forms easier to fabricate and less expensive. Finally, once the particles have been ingested and they imbibe water, they swell to a size which promotes retention, and they become exceptionally soft and slippery. As a consequence of the latter, they tend to resist expulsion from the stomach by the peristaltic motion of the stomach walls better than the particles of the prior patent.

Hydroxyalkylcelluloses have been used commercially as binders for sustained release tablets, and as ingredients in ophthalmic preparations.

The invention is a sustained-release oral drug dosage form for releasing a solution of a drug into the stomach comprising
a solid-state drug dispersed within non-crosslinked hydroxyethylcellulose particles that (i) swell unrestricted dimensionally via imbibition of gastric fluid to increase the size of the particles to promote maintenance of the "fed mode in the patient, with consequent increase in gastric retention time of the particles, and makes the particles slippery which also promotes their retention within the stomach, (ii) permits dissolution of the dispersed drug by imbibed gastric fluid while the drug is within the particle and release of the resulting solution, thus assuring that only drug in solution contacts the gastric mucosa, (iii) protects undissolved drug in the particles from stomach enzymes or pH effects so that undegraded drug is delivered to the stomach or duodenum, and (iv) maintains its physical integrity over at least a substantial portion of the time period during which the drug is released into the stomach and then dissolves. The dosage form is preferably in the form of a tablet or capsule that maintains the particles in a packed mass prior to their ingestion, the tablet or capsule then rapidly disintegrates in the gastric fluid to permit the particles to disperse in the stomach.

The dosage forms of the present invention are effective for administering drugs of limited solubility in gastric fluid that are capable of acting locally within the gastrointestinal tract or systemically by absorption into circulation via the gastrointestinal mucosa. The drug should be solid and not so water-soluble that it is rapidly leached from the particles over a very short time (i.e., less than about four hours), nor so insoluble that too little is leached from the particles to achieve the desired therapy. Thus, drugs having a solubility that permits them to dissolve and leach from the particles at a rate that provides the pharmacokinetics for therapy and the desired duration of treatment are selected. Normally, the solubility of the drug (measured in water at 37°C) will be in the range of 0.001% to about 35% by weight, more normally 0.001% to 5% by weight.

The invention is particularly useful for delivering drugs that are irritating to the gastrointestinal tract such as the mucosa of the stomach as a solid, drugs that are efficacious when administered in a sustained manner within the stomach, and drugs that are labile in the environment of the stomach. For instance, aspirin, which may be highly injurious to the gastric mucosa in its solid state, is advantageously administered in either high doses (generally 800 to 1400 mg over 10-14 hours) for analgesia or arthritis or at low doses (usually 20 to 100 mg, preferably about 30 mg) over a 4 to 14 hour period for prevention of heart attack and stroke, reduced risk of colon or rectal cancer, prevention of migraine, or prevention of pregnancy-induced hypertension. Irritation is avoided or limited because the initially solid drug is slowly released in solution and also because the drug-containing particles are dispersed, thereby limiting the concentration drug at any one site. The controlled delivery of the present particles allows for treatment with less total amount of drug, which further reduces the irritation effect.

Drugs which are effective for eradicating *Helicobacter pylori* from the submucosal tissue of the gastrointestinal tract, particularly the stomach, to treat stomach and duodenal ulcers, treat gastritis and esophagitis, and reduce risk of gastric carcinoma may also be administered effectively via the invention because the invention provides enhanced gastric retention and prolonged release. Drugs and drug combinations suggested for this indication include bismuth salts such as bismuth subsalicylate and bismuth citrate, metronidazole, and amoxycillin, other antibiotics such as tetracycline, neomycin or erythromycin, or combinations of such drugs. Preferred drugs for this indication are a bismuth salt plus metronidazole, amoxycillin plus metronidazole, and amoxycillin or a bismuth salt plus omeprazole.

Alternatively, the invention can be used with conventional ulcer treating drugs such as an H-2 antagonist (e.g., cimetidine or ranitidine) or an antacid such as calcium carbonate. In this regard, some agents appear to be more effective in an anacidic stomach; hence the presence of such acid reducing agents may be desirable.

Drugs such as peptides and proteins which are labile to the effects of gastric pH or gastric enzymes may also be effectively administered via the invention because the undissolved portion of the drug is physically protected within the particle until its dissolution and release, allowing for continuous delivery of undegraded drug at or near the site for the most efficient absorption of many such drugs -- e.g., from the lower stomach through the duodenum to the upper part of the small intestine which is the site within the gastrointestinal tract for the most efficient absorption of many molecules which are too large for significant absorption elsewhere. The ultimate advantage of this feature is that it allows for the oral administration of some therapeutic agents which otherwise require administration by injection. Examples of such agents are calcitonin, calcitriol and insulin.

Another example is that group of drugs known as proton pump inhibitors, such as omeprazole, which benefit from the slow release to optimize absorption while being protected from gastric acid.

This feature also allows for enhanced opportunity for bioabsorption of therapeutic agents which, while they may be absorbed to some extent from the G.I. tract, they are not under normal circumstances efficiently absorbed. Examples of such agents are cyclosporins, acyclovir, cephalosporins, interleukins, nitrofurantoin, and the ergot alkaloids.

Since it provides drug by continuous delivery instead of the pulse-entry associated with conventional dosage forms, two particularly significant benefits obtained with the present invention are: (1) The reduction in side effects from the drug, and (2) The ability to effect treatment with less frequent administration of the drug(s) being used. The following drugs when formulated in accordance with the invention provide these advantages, as well as other advantages as noted: Reduction in the side effects of angioedema, and agranulocytoses from angiotensin converting enzyme inhibitors such as elanopril maleate and captopril; reduction of anti-cholinergic (drying) and sedative side effects while providing long-lasting desired effects of antihistamines, such as clemastine fumarate; prolonged activity through gastric retention, less frequent administration requirements, and reduced side effects such as liver dysfunction, rhabdomyolysis, rash and headache, from cholesterol lowering drugs such as lovostatin; provision of more prolonged effects of antidepressant agents such as fluoxetine, with a reduction of typical side effects of these agents, such as insomnia and stomach upset; reduction in the required administration from three or four times daily to once daily, and reduction of the side effects, of antiepileptic drugs such as carbamazepine; and steady, prolonged control of pain, with reduced drug toxicity, from potent analgesics such as meperidine are obtained.

Benefits by way of reduction of the level of irritation and elevated LDL cholesterol may be obtained through use of formulations of this invention with blood platelet aggregation inhibitors such as ticlopidine.

A variety of similar benefits may be obtained with other types of drugs. Thus, provision, via controlled sustained delivery and gastric retention, of medication prolonged sufficiently to extend through the night so as to alleviate early morning hypertension, the cause of many heart attacks; and also reduction in the required frequency of administration to once daily dosing; of calcium channel blockers, such as verapamil, diltiazem, nifedipine, or nicardipine are obtained. Use of the invention provides, via gastric retention of the system, for more effective utilization of gastrointestinal prokinetic agents such as cisapride. The invention also enhances the treatment of gastroesophageal reflux disease by providing prolonged, local effects of agents that improve the competency of lower esophageal sphincter (LES) muscles. Such agents, which act directly on the LES muscles, include pentagastrin, PG-F2, and metaclopramide.

Other drugs that may be advantageously administered via the invention include, without limitation, H-2 antagonists or calcium carbonate for ulcer treatment/prevention; non-steroidal anti-inflammatory agents (NSAIDS) such as indomethacin, ibuprofen, naproxen and piroxican; steroids such as prednisone, prednisolone and dexamethasone; other NSAIDS such as diclofenac and ketorolac; acyclovir for the treatment of viral diseases such as herpes; tamoxifen for treatment of cancer; chlorpheniramine maleate for allergic disorders; potassium chloride for potassium supplementation, and peptides or other labile molecules such as protease inhibitors for treating AIDS.

The solid drug or drugs are dispersed in the hydroxyethylcellulose which ultimately dissolve in the gastrointestinal (G.I.) tract in a predictably delayed manner. The hydrophilicity and water swellability of these polymers cause the drug-polymer particles to swell in size, become slippery, and in the gastric cavity permit the ingress of water into the particle. The release rate of the drug(s) from the particles is primarily dependent upon the rate at which the drug(s) is leached from the particles, which in turn is related to the dissolution rate of the drug, the particle size and drug concentration in the particle. Correlatively, because these polymers dissolve very slowly in gastric fluid, the particles maintain their integrity over at least a substantial portion (i.e., at least about 90% and preferably over 100% of the intended dosing period). Thereafter the polymer will slowly dissolve. As indicated previously, such dissolution does not involve chemical degradation (i.e., cleavage of crosslinks) of the polymer and its dissolution is thus innocuous. Typically the polymer will have completely dissolved within 8 to 10 hours after the intended dosing period.

All alkyl-substituted cellulose derivatives in which the alkyl groups have 1 to 3 carbon atoms, preferably 2 carbon atoms, and having suitable properties as noted are contemplated. Cellulose is used herein to mean a linear polymer of anhydroglucose. Additional examples of suitable alkyl-substituted cellulose are: methylcellulose, hydroxymethylcellulose and carboxymethylcellulose. In general, suitable alkyl-substituted celluloses have a mean viscosity from 1,000 to 4,000 mPa s. (1% aqueous solution at 20°C). Hydroxyethylcellulose available from Aqualon Company (Wilmington, Delaware) referred to as Natrasol® 250HX, NF. It has a viscosity of a 1 percent solution at 20°C of from 1500 to 2500 mPa s. is selected when only one polymer is used.

The drug/polymer mixture is in the form of a plurality of particles. The solid drug is preferably dispersed homogeneously in the polymer, although it need not be. The weight ratio of drug to polymer in the mixture or dispersion will normally be 1:4 to 2:1, preferably 1:2 to 1:1, and most preferably 2:3 to 1:1. The particles are preferably spherical in shape but may be in the shape of less regular, but equant, granules.

The swollen particles will be of a size that promotes their retention in the stomach when the patient is not in the fed mode (i.e., presence of food) and particularly when the patient is in the fed mode. This will normally be in the range of 2 to 15 mm, preferably 6 to 12 mm (measured as the diameter for spherical particles or largest dimension for irregularly shaped particles), but may be larger. Since the particles will typically swell up to twice their original diameter in about 40 minutes and three times their original diameter in about 5 hours, the initial particle size is usually in the range of 1 to 5 mm, preferably 2 to 4 mm. Because the particles retain their physical integrity during the dosing period, their swollen volume will remain substantially constant (i.e., typically less than a 25% decrease) over the dosing period.

For drugs not intended for gastric retention, a useful particle size range for the initial particles is .5 to 3 mm, with a preferred size of about 2 mm. Again, the swollen particles are about three times their initial diameter.

The particles may be formed into a packed mass for ingestion by conventional techniques. For instance, the particles may be encapsulated as a "hard-filled capsule" or a "soft-elastic capsule" using known encapsulating procedures and materials. The encapsulating material should be highly soluble so that the particles are rapidly dispersed in the stomach after the capsule is ingested. Alternatively and preferably, the particles may be mixed with tableting excipients compressed into a tablet or pill. Each unit dose, whether capsule or tablet, will preferably contain particles of a size which when swollen enhance the potential for gastric retention. With respect to the number of particles per unit dose, a useful quantity for addition to a size zero capsule is about 14 particles, preferably spheres of about 4 mm diameter, 37 spherical particles of about 3 mm diameter, or 115 spherical particles of about 2 mm diameter. A workable range of particles is 10-200 spherical particles in either a capsule or a tablet. In the preferred embodiment utilizing a tablet dosage form, the tablet contains, in addition to any inert matrix that may be utilized, from 5-20 spherical particles of a size range from 1 to 4 mm in diameter.

With respect to the inert matrix in the dosage form, in the preferred embodiment an ionizable swelling agent is included which functions to osmotically increase the rate and extent of water imbibition that occurs when the particles are exposed to gastric fluid. A variety of pharmaceutically acceptable substances which ionize are contemplated. Ionizable salts such as sodium chloride is one such material. Other inorganic salts that are pharmaceutically acceptable are potassium sulfate and magnesium sulfate. Ionizable organics, such as ionic polymers, for example, sodium carboxymethylcellulose, can also be used. The salt sodium bicarbonate is another example which is particularly useful in an antacid product. The increased swelling effect provided by the swelling agent enhances the propensity of the spheres to be retained in the gastric cavity. The addition of ionized salts also provides for increased rate of drug diffusion from the polymer matrix, which is a useful feature for drugs whose solubility is extremely low, and which otherwise could not be delivered from the system at a high enough rate to be useful.

As an example, it has been found that sodium chloride at a concentration of 10% by weight in a matrix of 45% aspirin/45% hydroxypropyl cellulose by weight doubles the rate of swelling of spheres made from this composition, compared to spheres devoid of sodium chloride, when the spheres are suspended in an aqueous medium; and the rate of aspirin delivery from such a system is approximately tripled. In the preferred embodiment, sodium chloride can be used for purposes of enhancing the swelling rate of polymeric spheres at a concentration range of from 1% to 5% by weight, preferably at a concentration of 1% or 2% by weight. This is a generally suitable range for swelling enhancing agents but any effective amount otherwise compatible with the drug and its use is contemplated.

An alternative to the preferred embodiment for enhancing sphere swelling properties involves addition of sodium starch glycolate, used in concentration of from 0.5% to 3%, and preferably at about a 1% concentration by weight.

As noted, in addition to an increase in the rate of sphere swelling caused by the ionizable swelling agent, the degree or extent of swelling is also increased. For example, where a 10% by weight of sodium chloride is incorporated in a particle of this invention, a 60% increase over the size of the sphere swollen without benefit of the salt inclusion is obtained.

Another additive for the inert matrix in the dosage form may be desirable when the selected drug is so soluble that it may be released at a rate more rapid than desired. Examples of such drugs are potassium chloride and various peptides used as pharmaceuticals. In order to reduce the rate of release of these high solubility drugs, the particles are formulated to include a long chain fatty acid ester of glycerin, such as glyceryl monooleate. As illustrated in the examples below, the glyceryl ester is first mixed with the selected drug and thereafter the drug/glyceryl ester combination is mixed with the cellulose polymer. In general, long chain fatty acid esters of glycerin in which the fatty acid moiety has 15 to 21 carbon atoms bonded to its carboxyl group are contemplated, with the monoester of glycerin being preferred. Both saturated and unsaturated fatty acids may be utilized in ester formation, including palmitic, stearic, oleic, linoleic and linolenic acids. In addition to glycerin monooleate, other preferred esters are glyceryl behenate and glyceryl monostearate. Suitable reduction in release rate of the drug is obtained by incorporating an effective amount of the selected glyceryl ester. In general, highly soluble drugs will exhibit the desired reduced release rate by adding .5 to 4 moles of the glyceryl ester for each mole of drug.

The particulate drug/polymer mixture may be made by a number of mixing and comminution techniques with the final particle being fabricated by one of the following five methods:
(1) Extrusion and spheronization, using for example a Luwa Corporation Extruder/Marumerizer, available from Luwa Corporation Process Division, Charlotte, North Carolina.
(2) Direct compression, using multicavity hemispherical punches and dies, available from Elizabeth Carbide Die Company, Inc., McKeesport, Pennsylvania. The punches and dies are fitted to a suitable rotary tableting press, such as the Elizabeth-Hata single-sided Hata Auto Press machine, with either 15, 18 or 22 stations, and available from Elizabeth Hata International, Inc., North Huntington, Pennsylvania.
(3) Injection or compression molding using suitable molds fitted to a compression unit, such as available from Cincinnati Milacron, Plastics Machinery Division, Batavia, Ohio.
(4) Rotogranulation, using equipment for this procedure available from Glatt Air Techniques, Inc., Ramsey, New Jersey.
(5) For non-spherical shapes, the method consists of the following steps: (a)compaction of the powder mix, (b) milling of the compacted mass, (c) selective sieving of the milled product, and (d) recycling the material not selected by the sieving process.

When direct compression is used as the manufacturing process to make spheres, the addition of lubricants may be helpful and sometimes very important to prevent "capping" of the particle when the pressure is relieved. This is increasingly important as smaller spheres or particles are made. Useful agents include magnesium stearate (in a concentration in the powder mix of from 1% to 8%, preferably about 3% by weight), and hydrogenated vegetable oil (about 1% to 5% by weight, preferably about 2% by weight). Hydrogenated vegetable oil is an NF (The National Formulary) substance comprising hydrogenated and refined triglycerides of stearic and palmitic acids.

Alternatively, capping may be eliminated with lower concentrations of the lubricants or other excipients if a unit shape is chosen part way between a sphere and a right cylinder. That is, the unit is a cylinder with convex, instead of flat, ends. Thus another embodiment of the invention is a plurality of pellets, instead of spheres, which are either prolate or oblate spheroids of approximately equant dimensions. That is, the diameter of the circular cross-section is near but is not equal to the length of the axis normal to the section. As with the sphere dimensions described elsewhere, this dimension is from 1 to 5 mm, and preferably 2-4 mm.

The dose of drugs from conventional medication forms is specified in terms of drug concentration and administration frequency. In sharp contrast, because it delivers a drug by continuous, controlled release, a dose of medication from the system described in the invention is specified by drug release rate, and by duration of the release. It is the continuous, controlled delivery feature of the system that allows for (a) reduced drug side effects, since only the level needed is provided to the patient, and (b) less need to provide for precision in the timing of repeated administrations.

Different drugs have different biological half-lives, which determine their required frequency of administration (once daily, four times daily, etc.). Thus, when two or more drugs are co-administered in one conventional medication unit, an unfavorable compromise is often required, resulting in an underdose of one drug and an overdose of the other. In an alternate dosage form of this invention, a plurality of drug-containing spheres are provided, some of the spheres containing a first drug/polymer composition, and designed to release its drug at its ideal rate and duration (dose), while other spheres may contain and release a second drug with the same or different polymer than used with the first drug at its ideal rate and duration which is different from the other drug. Control of the release rate of the differing drugs may also be obtained by combining different amounts of each of the drug/polymer particles in a common dosage form such as a tablet. For example, where two drugs are combined in a tablet made from 20 particles, 5 particles may contain one drug and 15 particles would contain the other drug.

Examples of drug combination products based on the invention are norethindrone plus ethinyl estradiol, a combination useful for fertility control, and acetaminophen plus codeine, a potent analgesic combination. In both examples, each single ingredient can be provided at its optimum release rate for optimum pharmacokinetics and biological activity.

This feature of the invention which allows for co-administration of physically separated drugs also allows for combination products which are otherwise impossible due to chemical incompatibility of the chosen drugs when formulated together.

### Example 1

### (This example does not represent the invention).

Aspirin tablets of the invention are manufactured according to the following four-step procedure:
(1) A combination of 40.0 g aspirin dry powder, 258.50 g dry hydroxypropylcellulose (HPC), and 1.50 g magnesium stearate is ground to 100 mesh (US Standard) and mixed in a suitable blender.
(2) The above mixture is compressed into essentially spherical pellets of 3 mm diameter, using a rotary press fitted with 3 mm hemispherical, landed punches. Except for minor lossage, this procedure will result in a total mass of 300 g, representing approximately 10,000 pellets, with an individual weight of 30 mg per pellet.
(3) A combination of 750 mg magnesium stearate, 15 g powdered corn starch, 80 g lactose and 54.25 g HPC, all previously dried, are blended together in a PK blender to assure even mixing of the uneven-sized ingredients, while protecting from moisture. This blend may be compressed into one-inch (2.54 cm) diameter, one-quarter inch (6.35mm) thick tablets, using a rotary tablet press fitted with punches and dies suitable for this size, and the tablets produced by this precompression ("slugging") procedure are milled in a suitable mill, and sized by sieving to produce a fraction of irregular surfaced, essentially equant granules of approximately 3 mm in cross section. Granules too fine or too course are recycled through the precompression, milling and sieving process in order to reduce waste.
(4) A combination of 300 g of pellets produced by step (2) and 150 g of granules produced by step (3) are directly compressed into 1.1 cm diameter, 4.0 mm thick tablets with slightly convex faces, using a rotary press and a tablet punch with slightly convex faces and a die volume set to accept 450 mg of this mixture.

Tablets so produced disintegrate within 20 minutes in the stomach following ingestion, with the release and dispersion of ten spherical pellets, which swell to a diameter of 10 mm within 30 minutes, facilitating gastric retention. The pellets collectively release 40 mg of aspirin into the gastrointestinal tract over a period of from 8 to 10 hours. During this time the aspirin is released in the solution state rather than the solid state. Moreover, the pellets disperse within the stomach. Both dispersion and solution-state delivery operate to reduce the G.I. irritation from the delivered aspirin.

### Example 2

The following ingredients are dried, ground and blended together in a "twin shell" blender for 210 minutes: 111.11 g metronidazole, 111.11 g bismuth subcitrate, 1.5 g magnesium stearate, and 76.28 g hydroxyethylcellulose. This mixture is compressed into spherical pellets 3 mm in diameter, using a rotary press fitted with 3 mm hemispherical dies. This procedure produces approximately 300 g of pellets, each weighing 30 mg (approximately 10,000 pellets). These pellets are filled into size zero gelatin capsules, with the result that each capsule will contain 36 pellets. Upon ingestion, such capsules disintegrate rapidly in the G.I. tract, allowing dispersal of the pellets, which then swell to facilitate gastric retention, and collectively deliver 400 mg of both metronidazole and bismuth subcitrate over a period of from 8 to 10 hours to eradicate ulcer-producing local organisms.

### Example 3

Example 5 is repeated except that the 111.11 g of bismuth subcitrate is replaced with a like amount of amoxycillin.

### Example 4

Example 1 is repeated in which the ingredients of step (1) are replaced by 40 g aspirin, 15 g sodium chloride, 243.5 g hydroxyethylcellulose, and 1.5 g magnesium stearate.

### Example 5

Example 4 is repeated in which 15 g of sodium chloride are replaced by a like amount of potassium sulfate.

### Example 6

Example 1 is repeated in which hydroxypropylcellulose is replaced by hydroxyethylcellulose.

### Example 7

Example 1 is repeated in which the mixture of step (1) is replaced by a mixture of 150 g of meperidine base and 150 g of hydroxypropyl methylcellulose, and step (2) is replaced by the extrusion/spheronization procedure of Example 1.

### Example 8

Example 7 is repeated in which the mixture of step (1) is replaced by a mixture of 100 g of carbamazepine USP and 200 g of hydroxyethylcellulose.

The final tablets produced allow for sustained anticonvulsive effects from once or twice daily administration, compared to three or four times daily administration required by conventional tablets, and also provide for reduced intensity of this drug's side effects of cardiovascular disorder, aplastic anemia, and erythematous rash.

### Example 9

Example 7 is repeated in which the mixture of step (1) is replaced by a mixture of 40 g of fluoxitine base and 260 g of hydroxyethylcellulose.

The final tablets produced allow for sustained antidepressant effects from once daily administration, compared to twice daily administration of conventional tablets, and also provide for reduced intensity of this drug's side effects of insomnia and upset stomach.

### Example 10

Example 7 is repeated in which the mixture of step (1) is replaced by a mixture of 40 g of lovostatin and 260 g of hydroxyethylcellulose.

The final tablets produced allow for sustained cholesterol-lowering effects of this drug from once daily administration, with reduced intensity of its gastrointestinal, musculoskeletal, and CNS side effects.

### Example 11

Example 7 is repeated in which the mixture of step (1) is replaced by a mixture of 20 g of omeprazole and 280 g of hydroxyethylcellulose.

The dosage form of the invention protects the reservoir of undelivered drug from acid degradation of omeprazole, which is an acid-labile drug.

### Example 12

Example 7 is repeated in which the mixture of step (1) is replaced by a mixture of 60 g of diltiazem and 240 g of hydroxyethylcellulose.

The final tablets produced disintegrate upon ingestion, releasing the spheres contained therein, which disperse and swell in the stomach, thus facilitating gastric retention of the system. Sustained effect of this cardiovascular drug through the night from once-daily, bedtime administration is also provided. Accordingly, patients are protected from early morning hypertension, the cause of many heart attacks.

### Example 13

Example 12 is repeated in which the mixture of step (1) is replaced by a mixture of 40 g of cisapride and 260 g of hydroxyethylcellulose.

The final tablets produced disintegrate upon ingestion, releasing the spheres containing therein, which disperse and swell in the stomach, thus facilitating gastric retention and sustained local effect of the drug. Sustained, local delivery of this prokinetic agent allows for more efficient treatment of esophageal reflux disease.

### Example 14

Example 7 is repeated in which the mixture of step (1) is replaced by a mixture of 500 mg calcitonin, 280 g of hydroxyethylcellulose, and 41.33 mg of glyceryl monooleate. In preparing the formulation, the calcitonin and the glyceryl monooleate are first mixed intimately, and this mixture is then added to and mixed with the hydroxyethylcellulose.

The final tablets produced disintegrate upon ingestion, releasing the spheres contained therein, which disperse and swell in the stomach, thus facilitating gastric retention of the system. Controlled delivery of this soluble drug is facilitated by the presence of glyceryl monooleate.

The delivery system of the invention protects calcitonin, a labile agent, from degradation effects of gastric acid and gastric enzymes while the system is retained in the stomach. Moreover, delivery of the agent is continuously provided from the system retained in the lower part of the stomach, through the duodenum, to the upper part of the small intestine, which is the most efficient site of absorption of molecules too large to be appreciably absorbed elsewhere. When delivered by this system, a sufficient amount of calcitonin, a large peptide hormone, is absorbed to be clinically useful from oral administration. Otherwise, this agent must be administered by injection.

### Example 15

Example 7 is repeated in which the mixture of step (1) is replaced by a mixture of 100 g of cyclosporin USP, an immunosuppressive agent, and 200 g of hydroxyethylcellulose.

The final tablets produced disintegrate upon ingestion, releasing the spheres contained therein, which disperse and swell in the stomach, thus facilitating gastric retention and, through extended time of tissue exposure, increasing the amount of absorption of this otherwise difficult-to-absorb drug. Further, by providing cyclospsorin through low level, continuous delivery, the adverse effects of hepatotoxicity, nephrotoxicity, and hypertension are reduced.

### Example 16

Sustained release antacid tablets of the invention utilizing calcium carbonate an the active ingredient are prepared as follows:
(1) A combination of 150 g of dry calcium carbonate, 130 g of hydroxyethylcellulose, and 2 g of magnesium stearate are ground to 100 mesh (US Standard) and mixed in a suitable blender.
(2) The mixture from step (1) is compressed into essentially spherical pellets of 4 mm diameter, using a rotary tablet press fitted with hemispherical-cavity, landed punches and dies. Except for minor losses, this procedure will produce about 282 g of total pellet mass, representing about 1200 pellets, each weighing about 235 mg.
(3) The pellets of step (2) are used as a feed for a gelatin capsule filling operation in which 12 pellets are filled into each size zero capsule.

These capsules, following oral administration, rapidly disintegrate with dispersion of the pellets, which rapidly swell to promote their retention in the gastric cavity, and collectively release therein a total of 1.5 Gm of calcium carbonate over a 6 to 8 hour time period. This sustained release of the antacid agent into the stomach allows for patients who suffer from nocturnal gastric hyperacidity and/or esophageal reflux disease to sleep through the night from a single bedtime medication of a locally active agent (i.e., no systemic side effects). Less attractive alternatives for such patients are either multiple administrations of a conventional medication during the night, a regimen which interrupts sleep, or the use of longer-acting agents for gastric acid reduction such as cimetidine or ranitidine, which act systemically and therefore have adverse side effects.

## Claims

1. A sustained release oral drug dosage form for releasing a solution of a drug into the stomach comprising a solid-state drug dispersed within hydroxyethylcellulose particles that (i) swell unrestrained dimensionally via imbibition of water from gastric fluid to increase the size of the particles to promote maintenance of the fed mode in the stomach with consequent increase in gastric retention time of the particles, and makes the particles slippery which also promotes their retention within the stomach, (ii) permits dissolution of the dispersed drug by imbibed gastric fluid while the drug is within the particles and release of the resulting solution, thus assuring that only drug in solution contacts the gastric mucosa, (iii) protects undissolved drug in the particles from stomach enzymes or pH effects so that undegraded drug is delivered to the stomach and duodenum, and (iv) maintains its physical integrity over at least a substantial portion of the time period during which the drug is released into the stomach and then dissolves.

2. The dosage form in accordance with claim 1 wherein the dosage form is in the form of a tablet or capsule that maintains the particles in a packed mass prior to their ingestion and then rapidly disintegrates in the gastric fluid to permit the particles to disperse in the stomach.

3. The dosage form in accordance with claim 1 wherein the drug is aspirin, the sustained drug delivery time period is 10-14 hours and the total dose of aspirin delivered is 800 to 1400 mg.

4. The dosage form in accordance with claim 1 wherein the drug is aspirin, the sustained time period is 4 to 14 hours and the dose of aspirin is 20 to 100 mg.

5. The dosage form in accordance with claim 1 wherein the drug is a *Helicobacter pylori* eradicant.

6. The dosage form in accordance with claim 5 wherein the eradicant is a bismuth salt, metronidazole, amoxycillin, or a combination thereof.

7. The dosage form in accordance with claim 5 wherein the eradicant is amoxycillin or a bismuth salt plus omiprazole, an H-2 antagonist, or an antacid.

8. The dosage form in accordance with claim 1 wherein the particles are 1-5 mm in diameter in maximum dimension.

9. The dosage form in accordance with claim 1 wherein the particles are 2-4 mm in diameter in maximum dimension.

10. The dosage form in accordance with claim 2 wherein the dosage form is a tablet, the particles are spherical and 1-5 mm in diameter, and number 5-20 in one tablet.

11. The dosage form in accordance with claim 2 wherein the dosage form is a capsule, the particles are spherical and 1-5 mm in diameter, and number 10-200 in one capsule.

12. The dosage form in accordance with claim 10 wherein the dosage form is a tablet, the particles are spherical and 2-4 mm in diameter.

13. The dosage form in accordance with claim 11 wherein the dosage form is a capsule, the particles are spherical and 2-4 mm in diameter.

14. The dosage form in accordance with claim 1 and including sufficient ionizable swelling agent to osmotically cause imbibition of water and thereby increase the rate and extent of swelling in water.

15. The dosage form in accordance with claim 14 wherein the swelling agent is sodium chloride and is present in the particles at a concentration of 1 to 5 percent by weight.

16. The dosage form in accordance with claim 2 wherein the particles in said tablet or capsule contain a first drug and wherein said tablet or capsule also includes particles containing a second drug which differs from said first drug dispersed within said hydroxyethylcellulose particles.

17. The dosage form in accordance with claim 16 wherein the number of said first drug particles differs from the number of said second drug particles, said numbers of particles being selected to provide the desired delivered dose of each of said first and second drugs.

18. The dosage form in accordance with claim 16 wherein said first drug particles comprise hydroxyethylcellulose and said second drug particles comprise a second cellulose polymer different from hydroxyethylcellulose, said second polymer being selected to provide the desired release rates of said second drug.

19. The dosage form in accordance with claim 1 wherein said drug has a release rate greater than desired because of its water solubility and including sufficient long chain fatty acid ester of glycerin to reduce the release rate of said drug to a lower rate.

20. The dosage form in accordance with claim 19 wherein the drug is potassium chloride.

21. The dosage form in accordance with claim 19 wherein the drug is a peptide.

22. The dosage form in accordance with claim 19 wherein the glyceryl ester is selected from glyceryl monooleate, glyceryl behenate and glyceryl monostearate, the selected ester/drug ratio being .5 to 4 moles of ester per mole of drug.

23. The dosage form in accordance with claim 1 wherein said drug is cisapride.

24. The dosage form in accordance with claim 1 wherein said drug is calcium carbonate.

25. The dosage form in accordance with claim 1 wherein said drug is bismuth subsalicylate.

26. The dosage form in accordance with claim 1 wherein said drug is naproxen.

## Patentansprüche

1. Oral-Depotarzneimitteldosierungsform zur Freisetzung einer Lösung eines Arzneimittels in den Magen, umfassend ein in Hydroxyethylcellulosepartikeln dispergiertes festes Arzneimittel, welche (i) durch Wasseraufnahme aus der Magenflüssigkeit dimensional unbeschränkt aufquillt, um die Größe der Partikel zu erhöhen, um das Aufrechterhalten des Zufuhrmodus im Magen mit daraus folgender Zunahme der Magenretentionszeit der Partikel zu fördern, und die Partikel schlüpfrig macht, was ebenfalls ihre Magenretentionszeit erhöht, (ii) eine Auflösung des dispergierten Arzneimittels durch aufgenommene Magenflüssigkeit, während das Arzneimittel sich in den Partikeln befindet, und die Freisetzung der resultierenden Lösung erlaubt, wobei sichergestellt wird, daß Arzneimittel nur in Lösung mit der Magenschleimhaut in Kontakt kommt, (iii) ungelöstes Arzneimittel in den Partikeln vor Magenenzymen oder Wirkungen des pH-Werts schützt, so daß unzersetztes Arzneimittel in den Magen und Zwölffingerdarm abgegeben wird, und (iv) seine physikalische Beständigkeit über mindestens einen wesentlichen Teil der Zeitdauer bewahrt, während der das Arzneimittel in den Magen freigesetzt wird und sich dann auflöst.

2. Dosierungsform nach Anspruch 1, worin die Dosierungsform in Form einer Tablette oder Kapsel vorliegt, welche die Partikel vor ihrer Ingestion in einer gepackten Masse hält und dann rasch in der Magenflüssigkeit disintegriert, um eine Dispersion der Partikel im Magen zu ermöglichen.

3. Dosierungsform nach Anspruch 1, worin das Arzneimittel Aspirin ist, die Depotarzneimittel-Abgabezeitdauer 10 bis 14 Stunden beträgt und die Gesamtdosis an abgegebenem Aspirin 800 bis 1400 mg beträgt.

4. Dosierungsform nach Anspruch 1, worin das Arzneimittel Aspirin ist, die Depotarzneimittel-Abgabezeitdauer 4 bis 14 Stunden beträgt und die Aspirindosis 20 bis 100 mg beträgt.

5. Dosierungsform nach Anspruch 1, worin das Arzneimittel einMittel zur Bekämpfung von *Helicobacter pylori* ist.

6. Dosierungsform nach Anspruch 5, worin das Bekämpfungsmittel ein Bismutsalz, Metronidazol, Amoxycillin oder eine Kombination davon ist.

7. Dosierungsform nach Anspruch 5, worin das Bekämpfungsmittel Amoxycillin oder ein Bismutsalz plus Omiprazol, ein H-2-Antagonist oder ein Antazidum ist.

8. Dosierungsform nach Anspruch 1, worin die Partikel in der maximalen Dimension einen Durchmesser von 1-5 mm aufweisen.

9. Dosierungsform nach Anspruch 1, worin die Partikel in der maximalen Dimension einen Durchmesser von 2-4 mm aufweisen.

10. Dosierungsform nach Anspruch 2, worin die Dosierungsform eine Tablette ist, die Partikel sphärisch sind und einen Durchmesser von 1-5 mm aufweisen und 5-20 in einer Tablette zählen.

11. Dosierungsform nach Anspruch 2, worin die Dosierungsform eine Kapsel ist, die Partikel sphärisch sind und einen Durchmesser von 1-5 mm aufweisen und 10-200 in einer Kapsel zählen.

12. Dosierungsform nach Anspruch 10, worin die Dosierungsform eine Tablette ist, die Partikel sphärisch sind und einen Durchmesser von 2-4 mm aufweisen.

13. Dosierungsform nach Anspruch 11, worin die Dosierungsform eine Kapsel ist, die Partikel sphärisch sind und einen Durchmesser von 2-4 mm aufweisen.

14. Dosierungsform nach Anspruch 1 und umfassend ausreichend ionisierbares Quellmittel, um osmotisch Wasseraufnahme zu verursachen und dadurch das Ausmaß des Quellens und die Quellgeschwindigkeit in Wasser zu erhöhen.

15. Dosierungsform nach Anspruch 14, worin das Quellmittel Natriumchlorid ist und in den Partikeln in einer Konzentration von 1 bis 5 Gewichtsprozent vorhanden ist.

16. Dosierungsform nach Anspruch 2, worin die Partikel in der Tablette oder Kapsel ein erstes Arzneimittel enthalten und worin die Tablette oder Kapsel ebenfalls Partikel umfaßt, die ein zweites Arzneimittel enthalten, welches von dem in den Hydroxyethylcellulosepartikeln dispergierten ersten Arzneimittel verschieden ist.

17. Dosierungsform nach Anspruch 16, worin die Anzahl der Partikel mit dem ersten Arzneimittel von der Anzahl der Partikel mit dem zweiten Arzneimittel verschieden ist, wobei die Anzahlen so ausgewählt werden, daß die gewünschte zugeführte Dosis jeweils des ersten und zweiten Arzneimittels errreicht wird.

18. Dosierungsform nach Anspruch 16, worin die Partikel mit dem ersten Arzneimittel Hydroxyethylcellulose umfassen und die Partikel mit dem zweiten Arzneimittel ein zweites Cellulosepolymer umfassen, welches von Hydroxyethylcellulose verschieden ist, wobei das zweite Polymer so ausgewählt ist, daß es die gewünschten Freisetzungsgeschwindigkeiten des zweiten Arzneimittels ergibt.

19. Dosierungsform nach Anspruch 1, worin das Arzneimittel aufgrund seiner Wasserlöslichkeit eine größere Freisetzungsgeschwindigkeit als gewünscht hat und ausreichend langkettigen Fettsäureester von Glycerin enthält, um die Freisetzungsgeschwindigkeit des Arzneimittels auf eine niedrigere Geschwindigkeit zu reduzieren.

20. Dosierungsform nach Anspruch 19, worin das Arzneimittel Kaliumchlorid ist.

21. Dosierungsform nach Anspruch 19, worin das Arzneimittel ein Peptid ist.

22. Dosierungsform nach Anspruch 19, worin der Glycerylester ausgewählt ist aus Glycerylmonooleat, Glycerylbehenat und Glycerylmonostearat, wobei das ausgewählter-Ester/Arzneimittel-Verhältnis 0,5 bis 4 Mol Ester pro Mol Arzneimittel ist.

23. Dosierungsform nach Anspruch 1, worin das Arzneimittel Cisaprid ist.

24. Dosierungsform nach Anspruch 1, worin das Arzneimittel Calciumcarbonat ist.

25. Dosierungsform nach Anspruch 1, worin das Arzneimittel Bismutsubsalicylat ist.

26. Dosierungsform nach Anspruch 1, worin das Arzneimittel Naproxen ist.

## Revendications

1. Forme posologique médicamenteuse orale à libération prolongée, destinée à libérer une solution d'un médicament dans l'estomac, comprenant un médicament à l'état solide dispersé dans des particules d'hydroxyéthylcellulose qui (i) gonflent sans limitation dimensionnelle en s'imbibant d'eau provenant du fluide gastrique, ce qui permet d'augmenter les dimensions des particules pour favoriser le maintien du mode d'introduction dans l'estomac avec augmentation résultante du temps de rétention gastrique des particules, et ce qui rend les particules glissantes avec pour effet de favoriser également leur rétention à l'intérieur de l'estomac, (ii) permettent la dissolution du médicament dispersé par le fluide gastrique d'imprégnation tandis que le médicament est à l'intérieur des particules et la libération de la solution résultante, ce qui garantit que seul le médicament en solution entre en contact avec la muqueuse gastrique, (iii) protègent le médicament non dissous dans les particules contre les enzymes de l'estomac ou des effets du pH de telle sorte que le médicament non dégradé soit délivré à l'estomac et au duodénum, et (iv) maintiennent leur intégrité physique au moins pendant une partie importante de la période de temps pendant laquelle le médicament est libéré dans l'estomac et ensuite se dissout.

2. Forme posologique suivant la revendication 1, qui est sous forme d'un comprimé ou d'une capsule qui maintient les particules dans une masse tassée avant leur ingestion et qui se dissocie ensuite rapidement dans le fluide gastrique en permettant à des particules de se disperser dans l'estomac.

3. Forme posologique suivant la revendication 1, dans laquelle le médicament est l'aspirine, le temps de libération prolongée du médicament va de 10 à 14 heures et la dose totale d'aspirine délivrée va de 800 à 1400 mg.

4. Forme posologique suivant la revendication 1, dans laquelle le médicament est l'aspirine, la période de libération prolongée va de 4 à 14 heures et la dose d'aspirine va de 20 à 100 mg.

5. Forme posologique suivant la revendication 1, dans laquelle le médicament est un agent d'éradication d'Helicobacter pylori.

6. Forme posologique suivant la revendication 5, dans laquelle l'agent d'éradication est un sel de bismuth, le métronidazole, l'amoxycilline ou une de leurs associations.

7. Forme posologique suivant la revendication 5, dans laquelle l'agent d'éradication est l'amoxycilline ou un sel de bismuth plus l'omiprazole, un antagoniste H-2 ou un antacide.

8. Forme posologique suivant la revendication 1, dans laquelle les particules ont un diamètre de 1 à 5 mm en tant que dimension maximale.

9. Forme posologique suivant la revendication 1, dans laquelle les particules ont un diamètre de 2 à 4 mm en tant que dimension maximale.

10. Forme posologique suivant la revendication 2, qui est un comprimé, dont les particules sont sphériques et ont un diamètre de 1 à 5 mm, en un nombre de 5 à 20 particules dans un comprimé.

11. Forme posologique suivant la revendication 2, qui est une capsule, dont les particules sont sphériques et ont un diamètre de 1 à 5 mm, en un nombre de 10 à 200 dans une capsule.

12. Forme posologique suivant la revendication 10, qui est un comprimé, dont les particules sont sphériques et ont un diamètre de 2 à 4 mm.

13. Forme posologique suivant la revendication 11, qui est une capsule, dont les particules sont sphériques et ont un diamètre de 2 à 4 mm.

14. Forme posologique suivant la revendication 1 et comprenant une quantité d'agent gonflant ionisable suffisante pour lui permettre, par un processus osmotique, de s'inhiber d'eau et augmenter ainsi la vitesse et l'ampleur du gonflement dans l'eau.

15. Forme posologique suivant la revendication 14, dans laquelle l'agent de gonflement est le chlorure de sodium et est présent dans les particules à une concentration de 1 à 5 % en poids.

16. Forme posologique suivant la revendication 2, dans laquelle les particules présentes dans le comprimé ou la capsule contiennent un premier médicament, et dans laquelle ledit comprimé ou ladite capsule comprend également des particules contenant un second médicament qui diffère dudit premier médicament dispersé à l'intérieur desdits particules d'hydroxyéthylcellulose.

17. Forme posologique suivant la revendication 16, dans lequel le nombre des premières particules de médicament diffère du nombre des secondes particules de médicament, lesdits nombres de particules étant choisis de manière à obtenir la dose délivrée désirée de chacun des premier et second médicaments.

18. Forme posologique suivant la revendication 16, dans laquelle les premières particules de médicament comprennent de l'hydroxyéthylcellulose et les secondes particules de médicament comprennent un second polymère de cellulose différent de l'hydroxyéthylcellulose, ledit second polymère étant choisi de manière à obtenir les vitesses de libération désirées dudit second médicament.

19. Forme posologique suivant la revendication 1, dans laquelle le médicament a une vitesse de libération supérieure à la valeur désirée en raison de son hydrosolubilité, et comprenant une quantité d'un ester d'acide gras a chaîne longue du glycérol suffisante pour réduire la vitesse de libération dudit médicament à une valeur inférieure.

20. Forme posologique suivant la revendication 19, dans laquelle le médicament est le chlorure de potassium.

21. Forme posologique suivant la revendication 19, dans laquelle le médicament est un peptide.

22. Forme posologique suivant la revendication 19, dans laquelle l'ester de glycéryle est choisi entre le mono-oléate de glycéryle, le béhénate de glycéryle et le monostéarate de glycéryle, le rapport ester/médicament choisi étant un rapport de 0,5 à 4 moles d'ester par mole de médicament.

23. Forme posologique suivant la revendication 1, dans laquelle le médicament est le cisapride.

24. Forme posologique suivant la revendication 1, dans laquelle le médicament est le carbonate de calcium.

25. Forme posologique suivant la revendication 1, dans laquelle le médicament est le sous-salicylate de bismuth.

26. Forme posologique suivant la revendication 1, dans laquelle le médicament est le naproxène.
